# EUROPEAN PATENT SPECIFICATION

(11) **EP 1 150 716 B1**
(45) Date of publication and mention of the grant of the patent: **28.04.2004**
(21) Application number: 00902654.3
(22) Date of filing: 03.02.2000
(51) Int. Cl.: A61K 47/48, A61K 9/16, A61K 38/17

(54) **PHARMACEUTICAL COMPOSITION OF HYDROPHOBICALLY MODIFIED HEDGEHOG PROTEINS AND THEIR USE**
PHARMAZEUTISCHE ZUSAMMENSETZUNG VON HYDROPHOB MODIFIZIERTEN HEDGEHOG-PROTEINEN UND DEREN VERWENDUNG
COMPOSITION PHARMACEUTIQUE DE PROTEINES HEDGEHOG RENDUES HYDROPHOBES ET LEUR UTILISATION

(30) Priority: 04.02.1999 EP 99101643
(43) Date of publication of application: 07.11.2001
(73) Proprietor: Curis, Inc., Cambridge, MA 02138 (US)
(72) Inventor: PAPADIMITRIOU, Apollon, D-83673 Bichl (DE); LANG, Kurt, D-82377 Penzberg (DE)
(74) Representative: VOSSIUS & PARTNER
(86) International application number: PCT/EP2000/000847
(87) International publication number: WO 2000/045848

(56) References cited:
- EP-A- 0 947 201
- EP-A- 0 953 576
- EP-A- 0 978 285
- WO-A-90/08551
- WO-A-94/08599
- PORTER ET AL: "Cholesterol modification of hedgehog signaling proteins in animal development" SCIENCE, vol. 274, no. 2, 11 October 1996 (1996-10-11), pages 255-259, XP002082038
- BLAKE PEPINSKI R ET AL: "Identification of a palmitic acid-modified form of human sonic hedgehog" JOURNAL OF BIOLOGICAL CHEMISTRY, vol. 273, no. 22, 29 May 1998 (1998-05-29), pages 14037-14045, XP002094956
- KIKUCHI A ET AL: "Pulsed dextran release from calcium-alginate gel beads" JOURNAL OF CONTROLLED RELEASE, vol. 47, no. 1, 7 July 1997 (1997-07-07), page 21-29 XP004091190
- ROBINSON C J: "Neurotrophic factors - novel therapeutics" TRENDS IN BIOTECHNOLOGY, vol. 14, no. 12, December 1996 (1996-12), page 451-452 XP004071034
- DOWNS E.C. ET AL: "Calcium alginate beads as a slow-release system for delivering angiogenic molecules in vivo and in vitro" J. CELL. PHYSIOL., 1992, VOL. 152, NO. 2, PAGE(S) 422-429, XP002072808
- GRAY, C. J. ET AL: "Retention of insulin in alginate gel beads" BIOTECHNOL. BIOENG., 1988, VOL. 31, NO. 6, PAGES 607-612, XP002072809
- Y. YANG ET AL.: "Relationship between dose, distance and time in Sonic Hedgehog-mediated regulation of anteroposterior polarity in the chick limb" DEVELOPMENT, vol. 124, 1997, pages 4393-4404, XP002072810 cited in the application
- A. L PEZ-MART NEZ ET AL: "Limb-patterning activity and restreicted posterior localization of the amino-terminal product of Sonic hedgehog cleavage" CURRENT BIOLOGY, vol. 5, no. 7, 1995, pages 791-796, XP002072811 cited in the application
- E. MART ET AL.: "Requirement of 19K form of Sonic hedgehog for induction of distinct ventral cell types in CNS explants" NATURE, vol. 375, 25 May 1995 (1995-05-25), pages 322-325, XP002072812 LONDON, GB cited in the application
- M.J. BITGOOD ET AL.: "Sertoli cell signaling by Desert hedgehog regulates the male germline" CURRENT BIOLOGY, vol. 6, no. 3, 1996, pages 298-304, XP002072813 cited in the application
- N. KINTO ET AL.: "Fibroblasts expressing Sonic hedgehog induce osteoblast differentiation and ectopic bone formation" FEBS LETTERS., vol. 404, 1997, pages 319-323, XP002072814 AMSTERDAM NL cited in the application
- PORTER ET AL: "Hedgehog patterning activity: Role of a lipophilic modification mediated by the carboxy-terminal autoprocessing domain" CELL, vol. 86, no. 4, 12 July 1996 (1996-07-12), pages 21-34, XP002082039
- HAMMERSCHMIDT M ET AL: "The world according to hedgehog" TRENDS IN GENETICS, vol. 13, no. 1, 1 January 1997 (1997-01-01), page 14-21 XP004015059
- FARESE R V ET AL: "Cholesterol metabolism and embryogenesis" TRENDS IN GENETICS, vol. 14, no. 3, 1 March 1998 (1998-03-01), page 115-120 XP004108610
- YOSHIHIRO KAWAGUCHI ET AL: "CONFORMATIONAL CHANGES OF HYALURONATES WITH PARTIAL PALMITOYLATION AND THE ADSORPTION STRUCTURES ON THE SURFACE OF OIL DROPLETS" CARBOHYDRATE POLYMERS, vol. 20, no. 3, 1 January 1993 (1993-01-01), pages 183-187, XP000364403
- YOSHIHIRO KAWAGUCHI ET AL: "THE EFFECTS OF POLYSACCHARIDE CHAIN-LENGTH IN COATING LIPOSOMES WITH PARTIAL PALMITOYL HYALURONATES" CARBOHYDRATE POLYMERS, vol. 18, no. 2, 1 January 1992 (1992-01-01), pages 139-142, XP000262518
- ILLUM L ET AL: "HYALURONIC ACID ESTER MICROSPHERES AS A NASAL DELIVERY SYSTEM FOR INSULIN" JOURNAL OF CONTROLLED RELEASE, vol. 29, no. 1/02, 1 February 1994 (1994-02-01), pages 133-141, XP000433658

## Description

The invention concerns a pharmaceutical composition of hydrophobically modified hedgehog proteins and their use, in particular for the local release of these proteins on bones or cartilage.

Hedgehog (hh) proteins are understood as a family of secreted signal proteins which are responsible for the formation of numerous structures in embryogenesis (J.C. Smith, Cell 76 (1994) 193 - 196, N. Perrimon, Cell 80 (1995) 517 - 520, C. Chiang et al., Nature 83 (1996) 407, M.J. Bitgood et al., Curr. Biol. 6 (1996) 296, A. Vortkamp et al., Science 273 (1996) 613, C.J. Lai et al., Development 121 (1995) 2349). During its biosynthesis a 20 kD N-terminal domain and a 25 kD C-terminal domain are obtained after cleavage of the signal sequence and autocatalytic cleavage. In its natural form the N-terminal domain is modified with cholesterol (J.A. Porter et al., Science 274 (1996) 255 - 259). In higher life-forms the hh family is composed of at least three members namely sonic, indian and desert hh (shh, Ihh, Dhh; M. Fietz et al., Development (Suppl.) (1994) 43 - 51). Differences in the activity of hedgehog proteins that were produced recombinantly were observed after production in prokaryotes and eukaryotes (M. Hynes et al., Neuron 15 (1995) 35 - 44 and T. Nakamura et al., Biochem. Biophys. Res. Comm. 237 (1997) 465 - 469).

Hynes et al. compare the activity of hh in the supernatant of transformed human embryonic kidney 293 cells (eukaryotic hh) with hh produced from E. coli and find a four-fold higher activity of hh from the supernatants of the kidney cell line. The reason for this increased activity has been discussed to be a potential additional accessory factor which is only expressed in eukaryotic cells, a post-translational modification, a different N-terminus since the hh isolated from E. coli contains 50 % of a hh form which carries two additional N-terminal amino acids (Gly-Ser) or is shortened by 5 - 6 amino acids, or a higher state of aggregation (e.g. by binding to nickel agarose beads).

Nakamura et al. compare the activity of shh in the supernatant of transformed chicken embryo fibroblasts with an shh fusion protein isolated from E. coli which still has an N-terminal polyhistidine part. The shh in the supernatant of the fibroblasts has a seven-fold higher activity than the purified E. coli protein with regard to stimulation of alkaline phosphatase (AP) in C3H10T ½ cells. The increased activity has been postulated to be due molecules such as for example bone morphogenetic proteins (BMPs) which are only present in the supernatant of eukaryotic cells and cause the stronger induction of AP.

Kinto et al., FEBS Letters, 404 (1997) 319 - 323 describe that fibroblasts which secrete hh induce ectopic bone formation in an i.m. implantation on collagen. Thus hedgehog proteins have an osteoinductive activity.

A process for the production of delivery systems for proteins with a delayed release using alginate is known from WO 90/08551. A two-phase system is formed in which the first phase contains a high concentration of the protein (saturated solution) and the second phase contains alginate. However, such a phase separation is difficult and complicated to carry out when producing pharmaceutical compositions in large amounts.

A pulsatile release of dextran from calcium alginate complexes is known from Kikuchi, A. et al., J. Controlled Release 47 (1997) 21-29. However, the coupling of hedgehog proteins to such complexes is not described by Kikuchi.

In Trends in Biotechnology 14 (1996) 451 - 452 Robinson, C.J. et al. describe the intraventricular implantation of alginate microspheres as a method for the local application of NGF or NGF-secreting cells. An application for hedgehog proteins is, however, not described.

In J.Cell.Physiol. 152 (1992) 422 - 429 Downs, E.C. et al describe the use of calcium alginate spheres as a delivery system for angiogenesis factors. However, the use of this method or this delivery system for hedgehog proteins is not described.

In Biotechnol. Bioeng. 31 (1988) 607 - 612 Crey, C.J. and J. Dowsett describe the use of calcium/zinc alginate spheres as a delivery system for insulin. However, an application of this process to produce delivery systems for hedgehog proteins is not disclosed.

It is known from Yang et al., Development 124 (1997) 4393-4404 that high local hedgehog concentrations must prevail over a period of at least 16 h at the site of action in the body for a pharmaceutically effective in vivo activity. The carrier system for this described by Yang et al. i.e. the hedgehog-loaded chromatography medium affigel CM, the Ni agarose described by Marti et al., in Nature 375 (1995) 322-325 or the Affigel blue used by Lopez-Martinez et al., in Curr.Biol. 5 (1995) 791-796 or the heparin agarose particles that they used are less suitable for a pharmaceutical application since they are immunogenic and can cause inflammatory reactions.

The inventors found that the biocompatible and biodegradable carrier collagen described by Kinto et al. for hh-expressing cells is also unsuitable for an optimal local pharmaceutical application of hedgehog proteins. It was found that when the collagen carriers are loaded with hedgehog proteins under physiological conditions (pH ca. 7 and in weak acids (up to pH 4.5)) the majority of the applied hedgehog protein is released from the matrix within minutes. When the loading is carried out under acidic conditions (below pH 4.5) a large amount of the applied hedgehog protein is denatured and bound irreversibly to the carrier.

A pharmaceutical composition of a hedgehog protein is described in EP-A 947201 which is characterized in that the hedgehog protein is bound to a hydrophilic carrier which is biocompatible wherein the carrier is a polymer which binds the hedgehog protein as a negatively-charged carrier due to ionic interactions, the hedgehog protein is not denatured during binding to the carrier, it contains at least 0.1 to 2 negatively-charged residues per monomer under neutral conditions, the charge is in the form of acidic groups, it has an average molecular weight of at least 50,000 Da and contains no agarose.

The object of the invention is to provide a pharmaceutical composition of a hydrophobically modified hedgehog protein containing a biocompatible carrier, wherein the carrier binds the hedgehog protein as an active folded structure and can release it locally in vivo in an active form in a delayed manner. Such formulations are particularly suitable for the repair of bone and cartilage defects and can also be used to repair neuronal defects or for a systemic delivery.

The object is achieved by a pharmaceutical composition of a hydrophobically modified hedgehog protein which is characterized in that this composition contains hydrophobically modified hedgehog proteins and a biodegradable protein or a proteolytic degradation product thereof as a carrier, wherein said biodegradable protein binds the hedgehog protein and releases the protein in a delayed manner.

It has surprisingly turned out that hydrophobically modified hedgehog proteins can be released in vivo from such a carrier in a reversible, active and delayed manner without causing immunogenic and/or inflammatory reactions in vivo. The carrier can be a solid carrier such as a sponge or an injectable carrier such as a gel. The preferred biodegradable carrier is collagen or the hydrolysis product gelatin. Other fibrous carrier proteins such as fibrin or elastin are also suitable and can be used as intact protein fibres, as solubilized protein or as partially hydrolysed protein.

Collagen is understood according to the invention as preferably soluble collagen, insoluble collagen (preferably cross-linked), atelo-collagen or gelatin. According to the invention the collagen can be present as a solid matrix (cross-linked or non-cross-linked lyophilisate or precipitate, as fibres, as a dispersion or as a gel). Recombinantly produced collagen as well as type I or type II collagen and mixtures thereof are particularly suitable. Collagen fibres can for example be prepared according to the British Patent No. 1204438. Soluble collagen can for example be prepared by processes as described in the British Patents No. 990276 and 1184502. The product which is prepared by these methods can be referred to as a microgel and contains a mixture of collagen in various forms of aggregation. Hydrolysed collagen can preferably be obtained by treating collagen with trypsin. This results in a polydisperse mixture of polypeptides with a molecular weight in the range between about 5000 and 70000. The collagen is preferably firstly denatured for example by heat treatment before treatment with trypsin.

The biodegradable carrier, preferably collagen, is preferably used as a sponge such as for example the collagen sponge Helistat™ from the Integra Life Sciences Company, USA.

A collagen is preferably used as the carrier matrix, particularly preferably as a soluble or insoluble carrier matrix. The carrier matrix is particularly preferably composed of a combination of the biodegradable carrier and an anionic polysaccharide, such as preferably hyaluronic acid (as well as chemically cross-linked forms thereof), chondroitin sulfate, polyvinyl sulfate, keratan sulfate, dextran sulfate, pectin, carrageenan and other hydrocolloids, sulfated alginate, dermatan sulfate, alginate, preferably calcium alginate or complexes of protein and polysaccharides such as those described for example in the US patent No. 4,614,794 (Fibracol™, Johnson and Johnson, USA) in which the percentage by weight of the charged polysaccharides is 10-50 %. An insoluble matrix in the sense of the invention means that the matrix does not substantially decompose or significantly dissolve in a neutral buffer solution in vitro within 10-20 hours at room temperature. In this connection it is preferred that the carrier used according to the invention contains less than 50 %, preferably less than 20 % and particularly preferably essentially no amount of a neutral polysaccharide. A combination of collagen with hyaluronic acid with a molecular weight of at least 10⁶ Dalton, in particular with a molecular weight of 4 x 10⁶ Dalton is particularly suitable.

A delayed release is understood according to the invention as a release of the hedgehog protein in a pharmacologically effective dose over a defined period of at least 14 hours. A pharmacologically effect is understood as a neurological effect on nerve cells, a chondrogenesis and/or chondroinduction and preferably osteogenesis and/or osteoinduction as described in Kinto et al., FEBS Letters, 404 (1997) 319-323 for bone induction, by Miao et al. in J.Neurosci. 17 (1997) 5891-5899 for the effect on nerve cells and by Stott et al. in J.Cell Sci. 110 (1997) 2691-2701 for cartilage cell induction.

An enzymatically degradable carrier is preferably used as the carrier which is degraded by enzymes (e.g. proteases) secreted from the cells on which the local in vivo application has occurred. However, the half life of the carrier should be at least 12 h but can be several weeks. If the carrier is composed of a polysaccharide, this carrier is preferably degraded by glycosidases and by hydrolases that are present in and secreted by the cell. However, such a biodegradability of the carrier is not necessary in every case8 If the release is carried out to treat osteoporosis or neuronal diseases, a biodegradability is unnecessary. However, such carriers are preferably poorly soluble under physiological conditions and are therefore absorbed by the body over a long period (several weeks to months).

Solutions of hedgehog proteins at high concentrations are required to produce carrier matrices that are coated with hedgehog proteins in such a manner that they exhibit an adequate pharmaceutical efficacy when applied locally. It has turned out that pharmaceutically suitable carriers coated with hedgehog protein should preferably contain a concentration of the hedgehog protein of 1 - 20 mg/ml carrier and more. Carriers are particularly advantageous which contain hedgehog proteins at a concentration of 10 mg/ml carrier (solution volume, gel volume or sponge volume) or more. Hedgehog proteins are inherently poorly soluble. A further subject matter of the invention is a process for the production of a carrier matrix coated with hedgehog protein which is characterized in that the carrier matrix is incubated with a hedgehog protein solution at a concentration of 3 mg/ml which contains arginine or argininium ions and the carrier matrix coated in this manner is isolated.

Such solutions are suitable for producing carrier matrices which contain hedgehog proteins in pharmaceutically effective concentrations and are suitable for pharmaceutical applications. Hence a further subject matter of the invention is a collagen carrier which contains 3 mg hydrophobically modified hedgehog protein or more, preferably 10 mg or more per ml carrier matrix and arginine or argininium ions (preferably argininium sulfate). The concentration of arginine is preferably between 10 and 500 mmol/l, preferably in the pH range between 6 and 8.

Activity within the sense of the invention is understood as the activity of alkaline phosphatase (stimulation of the expression of alkali phosphatase) which the polypeptide can induce in mammalian cells (activity in the alkaline phosphatase test). In this method a mouse fibroblast cell line is cultured in a medium which contains foetal calf serum. Subsequently sterile filtered sample is added, the cells are lysed after ca. 5 days and alkaline phosphatase is determined in the cell lysate by means of the cleavage of a chromogenic substrate (pNP, p-nitrophenol) (J. Asahina, Exp. Cell. Res. 222 (1996) 38 - 47 and T. Nakamura (1997)).

A hydrophobically modified (lipophilized) hedgehog protein is understood by the invention as a lipophilized secreted signal protein which is responsible for the formation of numerous structures in embryogenesis. Sonic, indian or desert hh are particularly preferably used (Fietz M. et al., Development (Suppl.) (1994) 43-51). The processed form (N-terminal mature domain) of sonic hh protein described in EMBL data bank under the No. L38518 is preferably used. Proteins of the hedgehog family exhibit a pronounced homology in their amino acid sequence which is why it is also preferable to express those nucleic acids which code for hedgehog proteins which are 80 % or more homologous with the above-mentioned sequence of sonic hedgehog protein.

Such a lipophilization is preferably achieved by chemical modification. Such a hedgehog conjugate preferably contains an additional polypeptide that is covalently bound (preferably at the C-terminus and/or N-terminus) and is composed of 10 - 30 preferably hydrophobic amino acids and/or those amino acids which form transmembrane helices. The additional polypeptide particularly preferably contains 2 - 12 lysines and/or arginines but no polyhistidine part that would be suitable for purifying the conjugate on a Ni chelate column. It is also preferable to covalently bind (preferably at the C-terminus and/or N-terminus) 1 - 4 aliphatic, saturated or unsaturated hydrocarbon residues with a chain length of 10 - 24 C atoms or steroids with a lipophilic (hydrophobic) action. Furthermore it is preferred to covalently couple hydrophobic thio compounds, such as in particular thiocholesterol and thioalkanes, thioalkenes, to hh proteins via a disulfide bridge formed oxidatively (preferably at the C-terminus and/or N-terminus and in this case on the N-terminal cysteine).

The protein is hydrophobized by such lipophilizing residues which improves its interaction with lipid membranes of eukaryotic cells, in particular of mammalian cells.

Consequently a lipophilized protein according to the invention is understood as a hydrophobized protein which has an increased surface hydrophobicity compared to an unmodified protein which increases its affinity for apolar molecules or amphiphiles. The increase in the degree of lipophilicity of the protein can be measured by the degree of integration in a lipid layer as described for example by Haque, Z. et al., J.Agric.Food Chem. 30 (1982), 481. Methods for the hydrophobic (lipophilizing) modification of proteins are for example described by Haque, Z. et al., J.Agric.Food Chem. 31 (1983) 1225-1230; Webb, R.J. et al., Biochemistry 37 (1998) 673-679; Hancock, J.F., Cell 63 (1990) 133-139; A Practical guide to membrane protein purification, Ed. G.v. Jagow, Hermann Schägger (1994), (chapter 16, pages 535-554).

Such hydrophobically modified hedgehog proteins are described for example in the European Patent Application 953 576.

The human sonic hedgehog precursor protein is composed of the amino acids 1 - 462 of the sequence described in the EMBL databank under No. L38518. The amino acids 1 - 23 represent the signal peptide, the amino acids 24 - 197 represent the mature signal domain, the amino acids 32 - 197 represent the signal domain shortened by eight amino acids and the amino acids 198 - 462 represent the autoprocessing C-terminal domain after autoproteolytic cleavage.

The pharmaceutical composition according to the invention contains a pharmacologically effective dose of the hh protein and can be administered locally. It is preferable to use the proteins according to the invention in combination with other proteins of the hedgehog family or bone growth factors such as bone morphogenetic proteins (BMPs) (Wozney et al., Cell.Mol.Biol. of Bone, Bone Morphogenetic Proteins and their Gene Expression (1993) Academic Press Inc., 131-167) or parathyroid hormones (Karablis et al., Genes and Development 8 (1994) 277-289) or insulin-like growth factors (IGF-I or II) or transforming growth factors (TGF-β).

The pharmaceutical composition is produced by incubating the lipophilized hedgehog protein with the carrier or with the carrier containing collagen at a pH value of 4.5 or more. The incubation is preferably carried out at a neutral pH value (pH 6-8) and preferably in a buffered solution. At pH 4.5 or higher pH values, the lipophilized hedgehog protein binds to the carrier by hydrophobic interactions. If the carrier matrix additionally contains a hydrophilic carrier which can bind proteins by means of ionic interactions (for example an anionic polysaccharide), the hedgehog protein can then also interact with this part of the carrier by means of ionic interactions. For this reason the ratio of collagen to the hydrophilic carrier moiety can vary over a wide range. However, it is preferable that the ratio of collagen moiety to hydrophilic carrier moiety is 1.5:1 or more.

Furthermore it is preferable for the production of the pharmaceutical composition to add auxiliary substances such as a sugar (mannitol, lactose, glucose, sucrose, trehalose, preferably 20-100 mg/ml) or amino acids such as glycine or arginine, oxidation inhibitors such as methionine as well as antioxidants such as EDTA, citrate, polyethylene glycol (1 - 10 % by weight), detergents, preferably non-ionic detergents (preferably 0.005 - 1 % by weight) such as polysorbates (Tween®20 or Tween®80) or polyoxyethylenes, anti-inflammatory agents, local anaesthetics, antibiotics and/or stabilizers such as lipids, fatty acids and glycerol.

In a further preferred embodiment a pharmaceutical composition of the hedgehog protein according to the invention containing suramin is preferred and this can be used advantageously.

The pharmaceutical composition can contain additional pharmaceutical auxiliary substances.

In a preferred embodiment the pharmaceutical composition contains the lipophilized hedgehog protein at a concentration of 0.1 - 100 mg/ml.

In a preferred embodiment the pharmaceutical composition additionally contains a pharmaceutically acceptable buffer which is biocompatible, preferably in the range between pH 4 and pH 10, particularly preferably in the range between pH 6 and 8. The pH value of the pharmaceutical composition should be advantageously greater than pH 4 in order to prevent denaturation and detachment of the zinc complexed in the hedgehog protein. The concentration of the buffer is preferably 1-500 mmol/l, in particular 5-150 mmol/l and particularly preferably 10-100 mmol/l. In a suitable embodiment 300 mmol/l potassium phosphate buffer, pH 6.0 or 300 mmol/l arginine chloride, 10 mM potassium phosphate, pH 6.0 is used as a buffer.

The following examples, publications and the figures further elucidate the invention, the protective scope of which results from the patent claims. The described methods are to be understood as examples which still describe the subject matter of the invention even after modifications.

### Description of the Figures:

- Fig. 1:: In vitro release of hydrophobically modified shh from a collagen matrix (Helistat™).
- Fig. 2:: In vitro release of dipalmityl-shh from a collagen/alginate matrix (Fibracol™).

### Examples

### Example 1

### In vitro release of hydrophobically modified hedgehog proteins from a collagen matrix (Helistat™)

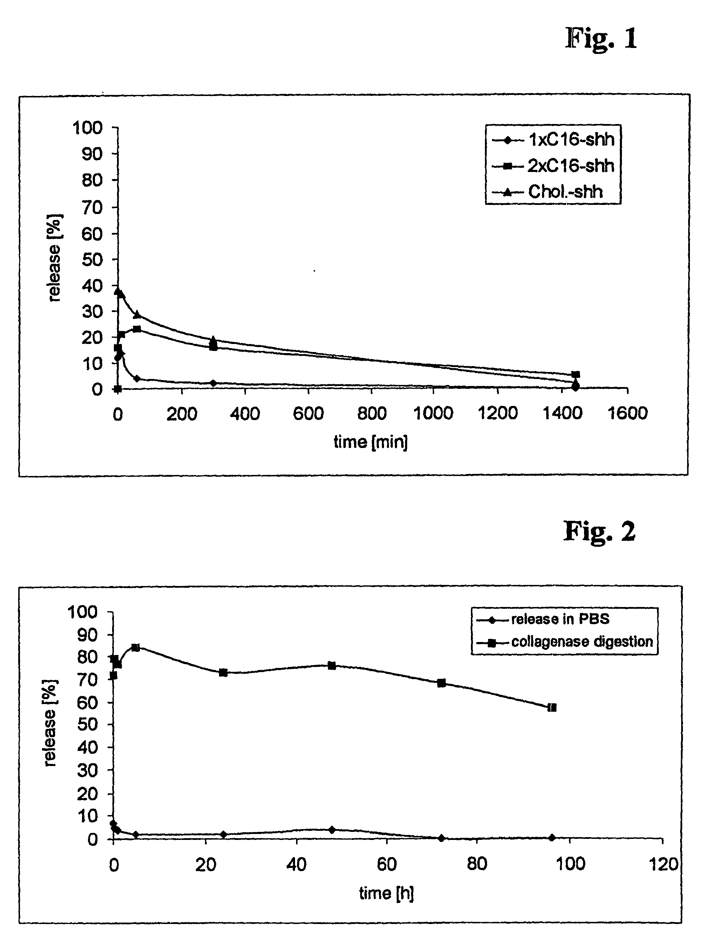

0.1 ml aliquots of solutions of variously modified hedgehog proteins (palmityl-shh = 1xC16-shh, dipalmityl-shh = 2xC16-shh or cholesteryl-shh = Chol-shh) are applied dropwise to collagen sponges (Helistat™, Integra Life Sciences, USA) with dimensions of 10 x 10 x 3 mm. The loaded carriers are frozen (-70°C), lyophilized and analysed. For this the sponges are incubated at 37°C in a suitable volume of PBS. The amount of released hh is determined by means of rp-HPLC (see Fig. 1).

The analysis of the in vitro release kinetics shows that Shh is released in a delayed manner or in small amounts from the collagen matrix over a period of 20 h (Fig. 1).

It can be seen that in vitro only ca. 20 % of the loaded 2xC16-shh is released under the stated conditions. The remaining 80 % of the applied protein can be released in vivo by biodegradation of the carrier. This situation can be simulated in vitro by an enzymatic degradation of the collagen carrier with recombinant collagenase. For this a collagen sponge loaded with 2xC16-shh which had been incubated for 14 h under release conditions (see above) was transferred into 490 µl buffer (50 mM HEPES-NaOH, 800 mM NaCl, 0.1 % Tween, pH 7.4) and 10 µl recombinant collagenase I (1 mg/ml, in 10 mM Tris-Cl, 1 mM CaCl₂, pH 7.4) was added. After incubation for 2 h at 37°C the released hedgehog protein is analysed by means of reversed phase HPLC and in an in vitro activity test (induction of alkaline phosphatase in C3H10T1/2 cells). It is clear from this analysis that the non-released fraction of 2xC16-shh (see above) that remains in the collagen carrier under release conditions can be released by means of collagenase digestion and its activity is not lost in this process (ca. 80 % recovery).

### Example 2:

### In vitro release of hydrophobically modified hedgehog protein (dipalmityl-shh) from a collagen-alginate matrix (Fibracol™)

0.05 ml aliquots of a solution of dipalmityl-shh (1 mg/ml) are added dropwise to collagen-alginate sponges (Fibracol™; Johnson & Johnson, USA) of approximately 10 x 10 x 3 mm size. The loaded carriers are frozen (-70°C), lyophilized and analysed. For this the sponges are incubated at 37°C in a suitable volume of PBS. The amount of released 2xC16-shh is determined by means of rp-HPLC (see Fig. 2).

It can be seen that in vitro only ca. 10 % of the loaded 2xC16 is released under the stated conditions. The remaining 90 % of the applied protein can be released in vivo by biodegradation of the carrier. This situation can be simulated in vitro by a partial enzymatic degradation of the carrier with recombinant collagenase. For this the carriers loaded with 2xC16-shh which had been incubated under release conditions (see above) were transferred into 490 µl buffer (50 mM HEPES-NaOH, 800 mM NaCl, 0.1 % Tween, pH 7.4) and 10 µl recombinant collagenase I (1 mg/ml, in 10 mM Tris-Cl, 1 mM CaCl₂, pH 7.4) was added. After incubation for 2 h at 37°C the released hedgehog protein is analysed after centrifugation by means of reversed phase HPLC. It is clear from this analysis that the non-released fraction of 2xC16-shh that remains in the collagen-alginate carrier under release conditions can be released by means of collagenase digestion (ca. 80 % recovery; Fig. 2).

### List of References

A Practical guide to membrane protein purification, Ed. G.v. Jagow, Hermann Schägger (1994), chapter 16, pages 535 - 554
Asahina, J., Exp. Cell. Res. 222 (1996) 38-47
Bitgood, M.J. et al., Curr. Biol. 6 (1996) 296
British Patent No. 1184502
British Patent No. 1204438
British Patent No. 990276
Chiang, C. et al., Nature 83 (1996) 407
Crey, C.J. et al., Biotechnol.Bioeng. 31 (1988) 607-612
Downs, E.C. et al., J.Cellular Physiology 152 (1992) 422-429
EP-A 98 101 893.0
European Patent Application No. 98107911.4
European Patent Application No. 98116733.1
Fietz, M. et al., Development (Suppl) (1994) 43-51
Hancock, J.F., Cell 63 (1990) 133-139
Haque, Z.et al., J. Agric.Food Chem. 30 (1982) 481
Haque, Z. et al., J. Agric.Food Chem. 31 (1983) 1225-1230
Hynes, M. et al., Neuron 15 (1995) 35-44
Karablis et al., Genes and Development 8 (1994) 277-289
Kikuchi, A. et al., J. Controlled Release 47 (1997) 21-29
Kinto et al., FEBS Letters, 404 (1997) 319-323
Lai, C.J. et al., Development 121 (1995) 2349
Lopez-Martinez et al. in Curr.Biol. 5 (1995) 791-796
Marti et al., Nature 375 (1995) 322-325
Miao et al., J. Neurosci. 17 (1997) 5891-5899
Nakamura, T. et al., Biochein. Biophys. Res. Comm. 237 (1997) 465-469
Perrimon, N., Cell 80 (1995) 517-520
Porter, J.A. et al., Science 274 (1996) 255-259
Robinson, C.J. et al., Trends in Biotechnology 14 (1996)
451-452
Smith, J.C., Cell 76 (1994) 193-196
Stott et al., J. Cell Sci. 110 (1997) 2691-2701
US-Patent 4,614,794
Vortkamp, A. et al., Science 273 (1996) 613
Webb, R.J. et al., Biochemistry 37 (1998) 673-679
WO 90/08551
Wozney et al., Cell. Mol. Biol. of Bone, Bone Morphogenetic Proteins and their Gene Expression, (1993) Academic Press Inc. 131-167
Yang et al., Development 124 (1997) 4393-4404

## Claims

1. A pharmaceutical composition containing a hydrophobically modified hedgehog protein and a biodegradable protein as a carrier, wherein said biodegradable protein binds the hedgehog protein and releases said protein in. a delayed manner.

2. The pharmaceutical composition of claim 1, containing soluble collagen as a carrier.

3. The pharmaceutical composition of claim 1, containing insoluble, cross-linked collagen.

4. The pharmaceutical composition of any one of claims 1 to 3, containing a hyaluronic acid or alginate.

5. The pharmaceutical composition of any one of claims 1 to 4, containing a hedgehog protein at a concentration of 0.1-100 mg/ml.

6. The pharmaceutical composition of any one of claims 1 to 5, wherein the composition is buffered in a range between pH 4.5 and 10.

7. The pharmaceutical composition of any one of claims 1 to 6, containing arginine or argininium ions.

8. A process for the production of a pharmaceutical composition, wherein a hydrophobically modified hedgehog protein is combined in a therapeutically effective amount with a biodegradable protein as a carrier, and wherein said biodegradable protein binds the hedgehog protein and releases said protein in a delayed manner.

9. A process for the production of an insoluble, biodegradable protein carrier matrix which contains a hydrophobically modified hedgehog protein, wherein the carrier matrix is incubated with a solution containing the hedgehog protein at a concentration of 3 mg/ml or more and arginine or argininium ions at a concentration of 10 mmol/l or more and the carrier matrix coated in this manner is isolated, and wherein said biodegradable protein binds the hedgehog protein and releases said protein in a delayed manner.

10. A process for the production of a delayed release composition according to claim 1, wherein a hydrophobically modified hedgehog protein is combined in a therapeutically effective amount with a biodegradable protein carrier.

## Patentansprüche

1. Arzneimittel, enthaltend ein hydrophob modifiziertes Hedgehog-Protein und ein biologisch abbaubares Protein als Träger, wobei das biologisch abbaubare Protein das Hedgehog-Protein bindet und das Protein in verzögerter Weise freisetzt.

2. Arzneimittel nach Anspruch 1, das lösliches Kollagen als Träger enthält.

3. Arzneimittel nach Anspruch 1, das unlösliches, vernetztes Kollagen enthält.

4. Arzneimittel nach einem der Ansprüche 1 bis 3, das eine Hyaluronsäure oder ein Alginat enthält.

5. Arzneimittel nach einem der Ansprüche 1 bis 4, das ein Hedgehog-Protein in einer Konzentration von 0,1 bis 100 mg/ml enthält.

6. Arzneimittel nach einem der Ansprüche 1 bis 5, wobei die Zusammensetzung in einem Bereich zwischen pH 4,5 und 10 gepuffert ist.

7. Arzneimittel nach einem der Ansprüche 1 bis 6, das Arginin oder Argininiumionen enthält.

8. Verfahren zur Herstellung eines Arzneimittels, wobei ein hydrophobisch modifiziertes Hedgehog-Protein in einer therapeutisch wirksamen Menge mit einem biologisch abbaubaren Protein als Träger kombiniert wird, und wobei das biologisch abbaubare Protein das Hedgehog-Protein bindet und das Protein in verzögerter Weise freisetzt.

9. Verfahren zur Herstellung einer unlöslichen, biologisch abbaubaren Proteinträgermatrix, die ein hydrophob modifiziertes Hedgehog-Protein enthält, wobei die Trägermatrix mit einer Lösung inkubiert wird, die das Hedgehog-Protein in einer Konzentration von 3 mg/ml oder mehr und Arginin oder Argininiumionen in einer Konzentration von 10 mmol/l oder mehr enthält und die in dieser Weise beschichtete Trägermatrix isoliert wird, und wobei das biologisch abbaubare Protein das Hedgehog-Protein bindet und das Protein in verzögerter Weise freisetzt.

10. Verfahren zur Herstellung einer Zusammensetzung mit verzögerter Freisetzung nach Anspruch 1, wobei ein hydrophobisch modifiziertes Hedgehog-Protein in einer therapeutisch wirksamen Menge mit einem biologisch abbaubaren Proteinträger kombiniert wird.

## Revendications

1. Composition pharmaceutique contenant une protéine hedgehog modifiée de manière à la rendre hydrophobe et une protéine biodégradable comme support, dans laquelle ladite protéine biodégradable se lie à la protéine hedgehog et libère ladite protéine d'une façon retardée.

2. Composition pharmaceutique selon la revendication 1, contenant du collagène soluble comme support.

3. Composition pharmaceutique selon la revendication 1, contenant du collagène insoluble réticulé.

4. Composition pharmaceutique selon l'une quelconque des revendications 1 à 3, contenant un acide hyaluronique ou un alginate.

5. Composition pharmaceutique selon l'une quelconque des revendications 1 à 4, contenant une protéine hedgehog à une concentration de 0,1-100 mg/ml.

6. Composition pharmaceutique selon l'une quelconque des revendications 1 à 5, dans laquelle la composition est tamponnée à un pH compris entre 4,5 et 10.

7. Composition pharmaceutique selon l'une quelconque des revendications 1 à 6, contenant de l'arginine ou des ions d'argininium.

8. Procédé pour la production d'une composition pharmaceutique, dans lequel une protéine hedgehog modifiée de manière à la rendre hydrophobe est combinée dans une proportion efficace sur le plan thérapeutique avec une protéine biodégradable en tant que support, et dans lequel ladite protéine biodégradable se lie à la protéine hedgehog et libère ladite protéine d'une façon retardée.

9. Procédé pour la production d'une matrice de support de protéines insolubles et biodégradables qui contient une protéine hedgehog modifiée de manière à la rendre hydrophobe, dans lequel la matrice de support est incubée avec une solution contenant la protéine hedgehog à une concentration de 3 mg/ml ou davantage et de l'arginine ou des ions d'argininium à une concentration de 10 mmol/l ou davantage et la matrice de support chargée de cette manière est isolée, et dans lequel ladite protéine biodégradable se lie à la protéine hedgehog et libère ladite protéine d'une façon retardée.

10. Procédé pour la production d'une composition à libération retardée selon la revendication 1, dans lequel une protéine hedgehog modifiée de manière à la rendre hydrophobe est combinée dans une proportion efficace sur le plan thérapeutique avec un support de protéines biodégradables.
